# EUROPEAN PATENT APPLICATION

(11) **EP 1 921 154 A1**
(43) Date of publication of application: **14.05.2008**
(21) Application number: 07000262.1
(22) Date of filing: 08.01.2007
(51) Int. Cl.: C12Q 1/00, G01N 27/30, G01N 33/487

(54) **Biosensor**

(30) Priority: 10.11.2006 KR 20060111229
(71) Applicant: Infopia Co., Ltd., Dongan-gu Anyang-si, Gyeonggi-do (KR)
(72) Inventor: Bae, Byeong-woo, Anyang-si Gyeonggi-do (KR); Lee, Sung-dong, Yeongcheon-si Gyeongsangbuk-do (KR); Suk, Hong-seong, Anyang-si Gyeonggi-do (KR); Yoo, Jin-a, Dongan-gu Gyeonggi-do (KR); Kim, Ji-su, Dongan-gu Anyang-si, Gyeonggi-do (KR); Yang, Jeong-yun, Gyeongsangnam-do (KR)
(74) Representative: Grünecker, Kinkeldey, Stockmair & Schwanhäusser Anwaltssozietät

(57) **Abstract**

A biosensor measuring an analyte contained in a sample includes a lower insulating substrate (100) having at least one electrode (111-113) and an enzyme reactant layer (120) which is formed on the electrode to react with the analyte; a spacer (600) which is attached to the lower substrate and an upper substrate and is provided to form a sample injection area (610) to guide the sample to the electrode through the enzyme reactant layer; and an upper insulating substrate (700) which faces the lower substrate and has an air discharge area (710) to discharge air which is absorbed together with the sample through the sample injection area, where the air discharge area is formed on a layer different from that of the sample injection area.

## Description

### CROSS REFERENCE TO RELATED APPLICATION

This application claims the benefit of and priority to Korean Patent Application No. 2006-111229, filed on November 10, 2006, which is hereby incorporated by reference for all purposes as if fully set forth herein.

### BACKGROUND OF THE INVENTION

### FIELD OF THE INVENTION

The present invention relates to a biosensor and, more particularly, to a biosensor which electrochemically detects an analyte contained in a bio sample.

### DISCUSSION OF THE BACKGROUND

In chemical or clinical aspect, it is very important to make a quantitative or qualitative analysis of an analyte contained in a bio sample. It is a typical example to measure blood glucose levels or cholesterol levels in diabetic patients. The diabetic patients need to take a measurement of their blood glucose levels at regular intervals. Recently, manufacturers have been bringing out blood glucose meters.

The present invention discloses a biosensor which can conveniently measure blood glucose levels and quickly absorb bio-samples.

A biosensor, such as a blood glucose sensor for the regulation of blood glucose level in diabetic patients, typically includes an electrode system having a plurality of electrodes formed on an insulating substrate by screen printing methods, and an enzyme reactant layer having hydrophilic polymer, oxidoreductase, and electron acceptor formed on the electrode system.

When a sample containing an analyte is dropped on the enzyme reactant layer of the biosensor and dissolves the enzyme reactant layer, the analyte reacts with an enzyme and is oxidized, causing the electron acceptor to be reduced. After the enzyme reaction is completed, the oxidation current is obtained when the electron acceptor is electrochemically oxidized. The oxidation current is measured with a measurement apparatus, thereby obtaining the concentration of the analyte.

The measurement time of the biosensor depends on the absorption rate of the sample which is absorbed into the enzyme reactant layer. The absorption rate of the sample depends on the discharge rate of air which is present on the enzyme reactant layer.

Korean Patent Application Publication No. 2003-0004933, published on January 15, 2003, discloses a biosensor in which a spacer is provided such that a sample injection area and an air discharge area are formed in T-shape.

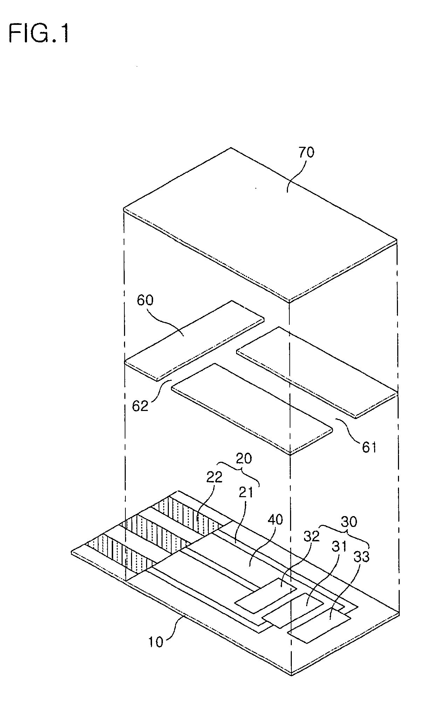

FIG. 1 is an exploded perspective view of a biosensor which is disclosed in the above-mentioned Korean Patent Application Publication No. 2003-0004933. The biosensor has a sample injection area 61 and an air discharge area 62, which are unitarily formed in T-shape. Since air is discharged through an enzyme reactant layer, the discharge rate of the air depends on the injection rate of the sample, causing the absorption rate of the sample to decrease. In addition, since the air discharge area needs to be large to increase the absorption rate of the sample, the amount of the sample is also large. Further, since the spacer 60 needs to be provided such that the sample injection area and the air discharge area are formed in T-shape, it becomes complicated to manufacture the biosensor.

Korean Patent Application Publication No. 2004-0028437, published on April 3, 2004, discloses a biosensor in which a slit is formed to discharge air in a direction parallel to an enzyme reactant layer from a recess formed on a cover to inject a sample.

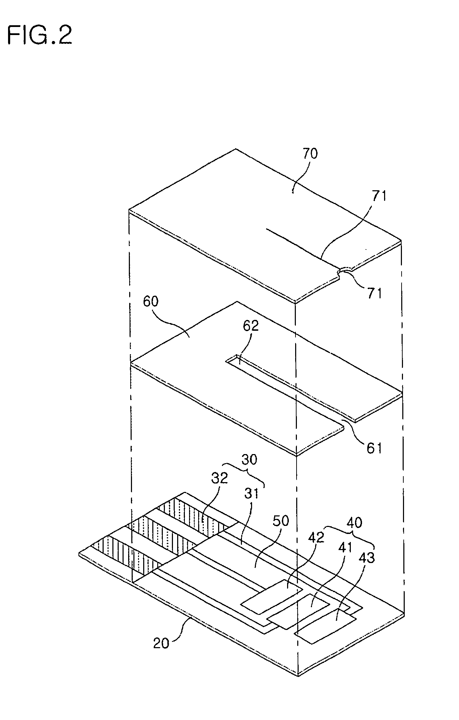

FIG. 2 is an exploded perspective view of a biosensor which is disclosed in the above-mentioned Korean Patent Application Publication No. 2004-0028437. Since the biosensor discharges the air through the recess formed on the upper substrate, the enzyme reactant layer may be contaminated, the sample may flow out through the recess and be smeared on a user's hands, the absorption amount of the sample is not constant, and the absorption rate of the sample is low since the recess cannot become unlimitedly large in size.

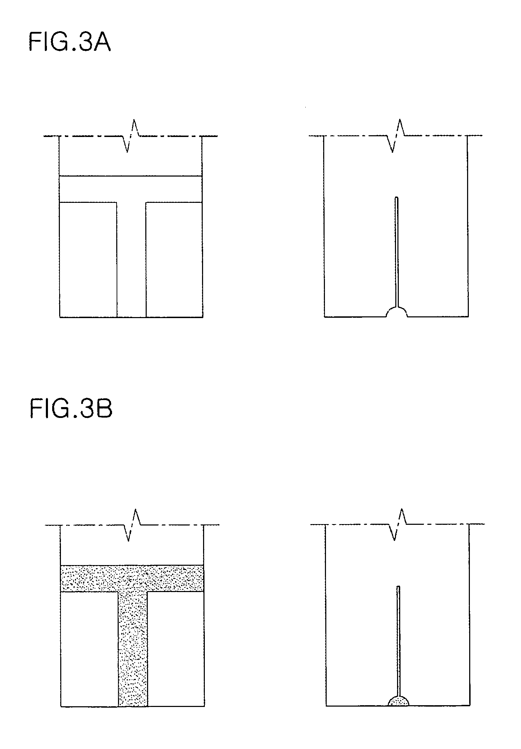

FIGS. 3A to 3C are views for explaining problems of the biosensors disclosed in the above-mentioned Korean Patent Application Publications. FIG. 3A shows a biosensor before a sample is injected into the biosensor. FIG. 3B shows a biosensor after the sample is injected into the biosensor. It is preferable that the sample is injected to an electrode through a sample inlet and does not flow out to an air discharge area.

However, as shown in FIG. 3C, the biosensors disclosed in the above-mentioned Korean Patent Application Publications have a problem in that the sample flows out towards the air discharge area. Accordingly, the user's hands may be smeared with the sample which flows out of the biosensor through the air discharge area. In addition, the absorption amount of the sample is not constant, resulting in unstable measurement results. Further, a more amount of the sample is needed for measurement.

Accordingly, a biosensor is required which has a wider air discharge area to increase the absorption rate of the sample and also minimizes the amount of the sample used in measurement. Further, a biosensor is required which quickly discharges air and prevents the sample flowing out through the air discharge area.

### SUMMARY OF THE INVENTION

The present invention provides a biosensor which increases the absorption rate of a sample, minimizes the amount of the sample used in measurement, and prevents the sample flowing out through the air discharge area.

Additional features of the invention will be set forth in the description which follows, and in part will be apparent from the description, or may be learned by practice of the invention.

The present invention discloses a biosensor measuring an analyte contained in a sample, including: a lower insulating substrate having at least one electrode and an enzyme reactant layer which is formed on the electrode to react with the analyte; a spacer which is attached to the lower substrate and an upper substrate and is provided to form a sample injection area to guide the sample to the electrode through the enzyme reactant layer; and an upper insulating substrate which faces the lower substrate and has an air discharge area to discharge air which is absorbed together with the sample through the sample injection area, where the air discharge area is formed on a layer different from that of the sample injection area.

The air discharge area may be formed in a tunnel shape on a layer different from that of the sample injection area to increase the absorption rate of the sample.

The air discharge area may be formed in a semi-cylindrical shape in a direction perpendicular to the absorption direction of the sample.

The cross-section of the air discharge area formed in a tunnel shape may have a width of about 0.3 to 3mm and a height of about 0.1 to 3mm, and the amount of the sample injected through the sample injection area may be less than about 1µl.

The sample injection area may grow narrower at an end portion where the sample injection area is adjacent to the air discharge area to prevent the sample from running out at the end portion of the sample injection area.

The spacer which contacts the lower substrate may be coated with hydrophobic adhesives and may be made of a hydrophobic material to prevent the sample from running out at an end portion of the sample injection area.

It is to be understood that both the foregoing general description and the following detailed description are exemplary and explanatory and are intended to provide further explanation of the invention as claimed.

### BRIEF DESCRIPTION OF THE DRAWINGS

The accompanying drawings, which are included to provide a further understanding of the invention and are incorporated in and constitute a part of this specification, illustrate embodiments of the invention, and together with the description serve to explain the principles of the invention.
FIGS. 1 and 2 are exploded perspective views of conventional biosensors.
FIGS. 3A to 3C are views for explaining problems of conventional biosensors.
FIG. 4 is an exploded perspective view of a biosensor according to an exemplary embodiment of the present invention.
FIG. 5 is a plan view of a biosensor according to an exemplary embodiment of the present invention.
FIG. 6 is a cross-sectional view of a biosensor according to an exemplary embodiment of the present invention.
FIGS. 7 and 8 are enlarged views of part of the biosensor shown in FIG. 6.
FIG. 9 is a view for explaining the effect of a biosensor according to an exemplary embodiment of the present invention.

### DETAILED DESCRIPTION OF THE ILLUSTRATED EMBODIMENTS

The invention is described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this disclosure is thorough, and will fully convey the scope of the invention to those skilled in the art. In the drawings, the size and relative sizes of layers and regions may be exaggerated for clarity. Like reference numerals in the drawings denote like elements.

It will be understood that when an element or layer is referred to as being "on" or "connected to" another element or layer, it can be directly on or directly connected to the other element or layer, or intervening elements or layers may be present. In contrast, when an element is referred to as being "directly on" or "directly connected to" another element or layer, there are no intervening elements or layers present.

A biosensor typically includes a working electrode and a reference electrode and/or a counter electrode. For example, an electrochemical sensor uses an oxidoreductase and an electron-transfer mediator to make a measurement based on the following reaction equation:

[Reaction equation] Analyte + Enzyme (oxidized) + Electron-transfer mediator (oxidized) → Product + Enzyme (reduced) + Electron-transfer mediator (reduced)

As expressed in the reaction equation, the reduced electron-transfer mediator which is produced from the reaction with the analyte contained in the sample is proportional to the concentration of the analyte. When a predetermined voltage is applied to the working electrode with respect to the reference electrode or counter electrode, the electron-transfer mediator is oxidized, thereby producing the oxidation current. At this time, the amount of the analyte contained in the sample can be determined from the oxidation current.

In this case, examples of the enzymes include oxidoreductase, such as glucose oxidase, lactate oxidase, cholesterol oxidase, and alcohol oxidase, transferase, such as glucose dehydrogenase, GOP (glutamate oxaloacetate transaminase, and GPT (glutamate pyruvate transaminase), and hydrolase.

Examples of the electron-transfer mediator include potassium ferricyanide, potassium ferrocyanide, hexaamineruthenium chloride, ferrocene and its derivative, and quinine and its derivative, which are substances reacting with the enzyme to be oxidized or reduced.

The electrode is made of carbon, graphite, platinum-plated carbon, silver, gold, palladium, or platinum. For example, an ink composed of carbon or platinum-plated carbon, or an ink containing palladium may be used to print an electrode on a lower substrate. Alternatively, the electrode may be formed through vacuum deposition of gold onto the lower substrate.

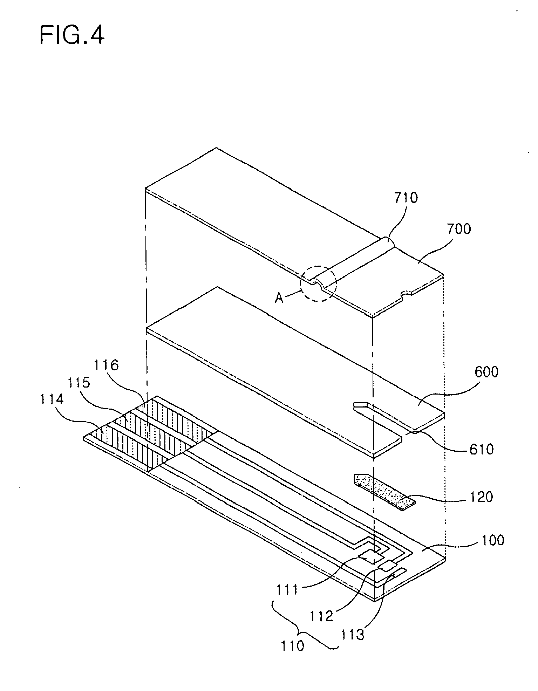

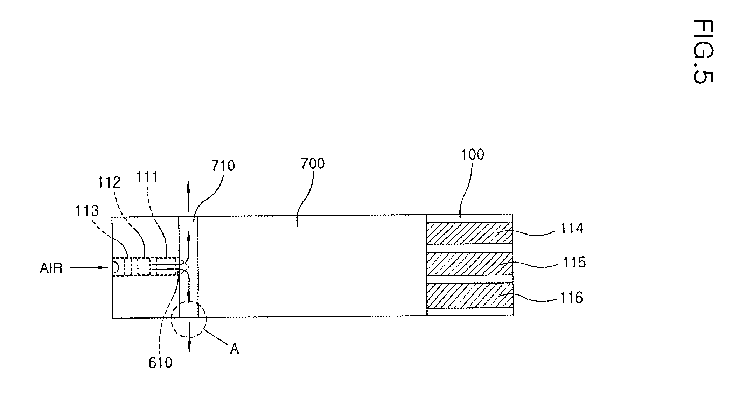

FIG. 4 is an exploded perspective view of a biosensor according to an exemplary embodiment of the invention. FIG. 5 is a plan view of a biosensor according to an exemplary embodiment of the invention.

The biosensor includes a lower insulating substrate 100, a spacer 600, and an upper insulating substrate 700.

The lower insulating substrate 100 has at least one electrode, and an enzyme reactant layer which is formed on the electrode to react with an analyte contained in a sample. The spacer 600 is interposed between the lower and upper substrates 100 and 700 and is provided such that a sample injection area 610 is formed. The sample injection area 610 is provided to guide the sample to the electrode through the enzyme reactant layer. The upper insulating substrate 700 faces the lower substrate 100 and has an air discharge area 710 to discharge air which is absorbed together with the sample through the sample injection area 610. The air discharge area 710 is formed on a layer different from that of the sample injection area 610.

The air discharge area 710 is preferably formed in a tunnel shape on a layer different from that of the sample injection area to increase the absorption rate of the sample. The air discharge area is more preferably formed in a semi-cylindrical shape in a direction perpendicular to an injection direction of the sample.

In more detail, the lower substrate 100 has at least one electrode 111 to 113 to measure the analyte contained in the sample, and an enzyme reactant layer 120 which is formed on the electrode to react with the analyte. The lower substrate 100 may be an insulating thin plate which is made of polyethylene terephtalate (PET), polyvinyl chloride (PVC) or polycarbonate. The lower substrate 100 may be made of an insulating material having a thickness of 50 to 400um, more preferably a thickness of 100 to 300um.

An electrode system having the electrodes 111 to 113 and leads 114 to 116 are printed on the lower substrate 100, in which the leads 114 to 116 contact a measurement apparatus (not shown), and the electrodes 111 to 113 are connected to the leads 114 to 116 to detect electrical signals flowing through the sample.

The enzyme reactant layer 120 includes an enzyme, which reacts with the analyte contained in the sample, an electron-transfer mediator, which reacts with the enzyme, and a polymer holding substance, which fixes a buffer solution, an enzyme stabilizer, and the like on the electrode. The enzyme reactant layer 120 is covered and fixed on the electrodes. When the sample containing the analyte is dropped on the enzyme reactant layer 120, the enzyme reactant layer 120 is dissolved by the sample and the analyte reacts with the enzyme, thereby oxidizing the analyte and reducing the electron acceptor. After the enzyme reaction, the oxidation current obtained by electrochemically oxidizing the reduced electron accepter is measured by a measurement apparatus (not shown) which contacts the leads 114 to 116 connected to the electrodes 111 to 113, thereby obtaining the concentration of the analyte contained in the sample.

The sample injection area 610 is formed by attaching the spacer 600 to the lower and upper substrates 100 and 700. The spacer 600 has a thickness not thinner than the enzyme reactant layer 120 in order to easily inject the sample to the enzyme reactant layer 120. That is, the sample is easily injected when the spacer 600 having a thickness greater than the enzyme reactant layer 120. The spacer 600 may be a double-sided adhesive tape with a thickness of 10 to 300um, more preferably a thickness of 10 to 150um, in order to minimize the amount of the injected sample. When the sample is automatically injected through the sample injection area 610 by capillary action, the air existing in the sample injection area 610 is discharged outside through the air discharge area 710 which is formed on the upper substrate 700.

The air discharge area 710 is preferably formed on a layer different from that of the sample injection area, more preferably in a tunnel shape, to increase the absorption rate of the sample. That is, the air discharge area 710 is formed on a layer on which the sample and the enzyme reactant layer 120 do not exist, such that only the air is discharged through the air discharge area 710. The air discharge area 710 is preferably formed in semi-cylindrical shape in a direction perpendicular to the absorption direction of the sample. The air discharge area 710 may be formed in other shapes.

When the sample starts to be injected to the enzyme reactant layer 120, the air existing around the enzyme reactant layer 120 is pushed toward the sample injection area 610, such that the air is discharged outside through the air discharge area 710 which is formed on the upper substrate 700 in a direction perpendicular to the injection direction of the sample. Since only the air is discharged outside through the air discharge area 710 which is formed in a tunnel shape on a layer different from that of the sample injection area 610, the absorption rate of the sample increases.

That is, since the air discharge area 710 is formed on a layer different from that of that of the enzyme reactant layer 120, the amount of the sample is irrelevant to the dimension of the air discharge area 710. Accordingly, the amount of the sample is as reduced as the dimension of the enzyme reactant layer 120. In addition, since the air discharge area 710 can be increased irrespective of the dimension of the enzyme reactant layer 120, it is possible to efficiently discharge the air. Further, since the air is discharged without passing through the enzyme reactant layer 120, the discharge rate of the air is irrelevant to the absorption rate of the sample. As a result, the discharge rate of the air increases, causing the absorption rate of the sample to increase. Further, when the biosensor is removed from the measurement apparatus, the sample is rarely smeared on a user's hands.

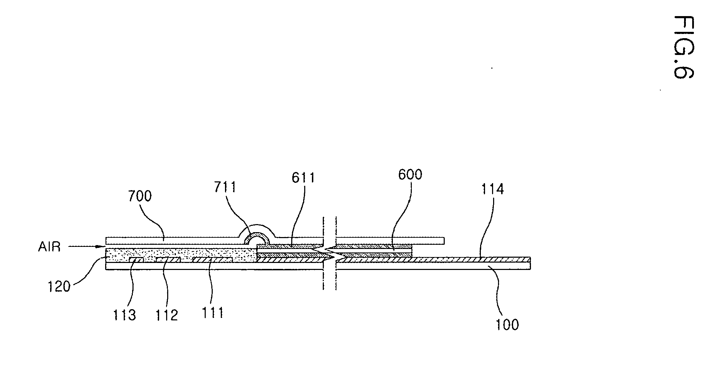

FIG. 6 is a cross-sectional view of a biosensor according to an exemplary embodiment of the invention.

The electrodes 111 to 113 and the lead 114 are formed on the lower substrate 100, and the enzyme reactant layer 120 is fixed on the electrodes. The lower substrate 100 is combined with the upper substrate 700 by the spacer 600. The air discharge area 710 is formed in a tunnel shape on the upper substrate 700 in a direction perpendicular to the injection direction of the sample to discharge the air when the sample is injected through the sample injection area 610.

The spacer 600 and the upper substrate 700 are preferably smaller in dimension than the lower substrate 100 so that a tip of the lead 114 printed on the lower substrate 100 can be exposed. The tip of the lead 114 contacts the measurement apparatus (not shown), and the measurement apparatus measures the oxidation current detected by the electrodes 111 to 113 to obtain the concentration of the analyte contained in the sample.

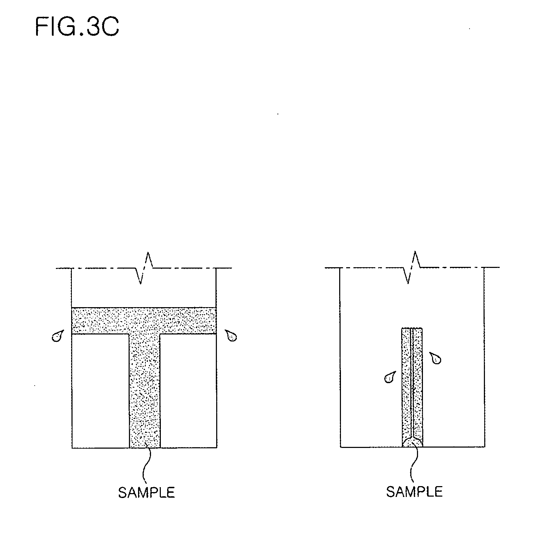

As shown in FIG. 3C, the conventional biosensor has a problem in that a sample spreads out towards an air discharge area. Therefore, the sample may flow out of the biosensor through the air discharge area and be smeared on a user's hands. In addition, the amount of the sample is not constant, resulting in unstable measurement values.

Accordingly, a biosensor is required which quickly discharges air through the air discharge area and prevents a sample from running out. The present invention proposes several solutions to prevent the sample from running out.

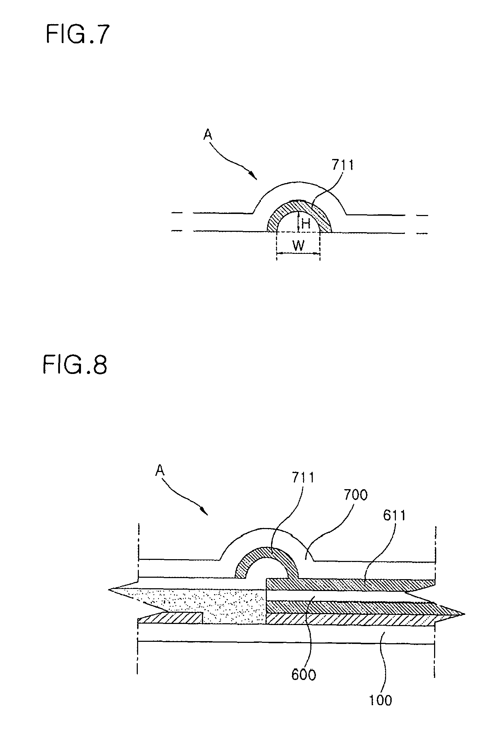

FIGS. 7 and 8 are enlarged views of part of the biosensor shown in FIG. 6.

As a first solution to prevent the sample from running out, the air discharge area 710 is limited in its width and height. In the event the cross-section of the air discharge area is too small, the sample may run out by a capillary action as shown in FIG. 3C. In the event the air discharge area 710 is large in its cross-section, it is possible to prevent the capillary action and quickly discharge the air. As a result, the absorption rate of the sample increases.

However, if the cross-section of the air discharge area 710 is too large, it is difficult to form the air discharge area 710 in a tunnel shape. In addition, the air discharge area 710 may be deformed. Thus, the air discharge area 710 needs to be limited in dimension.

According to experiments, the cross-section of the air discharge area 710 preferably has a width of about 0.3 to 3mm and a height of about 0.1 to 3mm. The amount of the sample injected through the sample injection area is preferably less than about 1µl.

As a second solution to prevent the sample from running out, the inner wall of the air discharge area 710 is coated with a hydrophobic material since the sample and the enzyme reactant layer 120 have a hydrophilic property.

As shown in FIGS. 7 and 8, the inner wall of the air discharge area which is formed in a tunnel shape is preferably coated with a hydrophobic material to prevent the sample from running out towards the air discharge area 710. The spacer 600 is preferably made of a hydrophobic material to prevent the sample from running out. Further, the spacer 600 is preferably attached to the upper and lower substrates with hydrophobic adhesives.

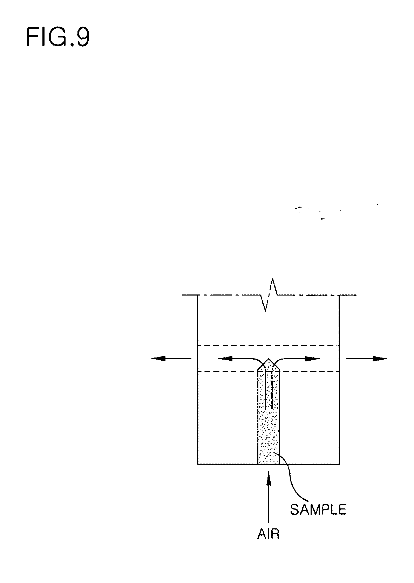

As a third solution to prevent the sample from running out, the sample injection area 610 is formed to become narrower at its end portion. As shown in FIG. 9, the sample injection area 610 preferably grows narrower at its end portion where the sample injection area 610 is adjacent to the air discharge area 710.

A sample injection hole may be further formed at a sample inlet of the upper substrate 700 to easily inject the sample. In this case, the enzyme reactant layer 120 will be rarely contaminated by the sample injection hole if the sample injection hole is formed to be very small.

FIG. 9 is a view for explaining the effect of a biosensor according to an exemplary embodiment of the present invention.

Referring to FIG. 9, a sample does not run out of the sample injection area 610, thereby minimizing the amount of the sample. At this time, only the air is quickly discharged through the air discharge area 710 which is formed on a layer different from that of the sample injection area 610.

As apparent from the above description, the biosensor is provided which can increase the absorption rate of a sample, minimize the amount of the sample used in measurement, and prevent the sample flowing out through the air discharge area.

That is, since the biosensor has the air discharge area which is formed on a layer different from that of the sample injection area in a direction perpendicular to a sample injection direction, it is possible to increase the sample absorption rate by efficiently discharging the air upon absorbing the sample. In addition, it is possible to prevent the sample running out of the sample injection area.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

## Claims

1. A biosensor measuring an analyte contained in a sample, comprising:
a lower insulating substrate having at least one electrode and an enzyme reactant layer which is formed on the electrode to react with the analyte;
a spacer which is attached to the lower substrate and an upper substrate and is provided to form a sample injection area to guide the sample to the electrode through the enzyme reactant layer; and
an upper insulating substrate which faces the lower substrate and has an air discharge area to discharge air which is absorbed together with the sample through the sample injection area,
wherein the air discharge area is formed on a layer different from that of the sample injection area.

2. The biosensor of claim 1, wherein the air discharge area is formed in a tunnel shape on a layer different from that of the sample injection area to increase the absorption rate of the sample.

3. The biosensor of claim 1, wherein the air discharge area is formed in a semi-cylindrical shape in a direction perpendicular to the absorption direction of the sample.

4. The biosensor of claim 1, wherein the cross-section of the air discharge area formed in a tunnel shape has a width of about 0.3 to 3mm and a height of about 0.1 to 3mm, and the amount of the sample injected through the sample injection area is less than about 1µl.

5. The biosensor of claim 1, wherein the sample injection area grows narrower at an end portion where the sample injection area is adjacent to the air discharge area to prevent the sample from running out at the end portion of the sample injection area.

6. The biosensor of claim 1, wherein an inner wall of the air discharge area is coated with a hydrophobic material to prevent the sample from running out at an end portion of the sample injection area.

7. The biosensor of claim 1, wherein at least one side of the spacer which contacts the upper substrate is coated with hydrophobic adhesives to prevent the sample from running out at an end portion of the sample injection area.

8. The biosensor of claim 1, wherein the spacer which contacts the lower substrate is coated with hydrophobic adhesives to prevent the sample from running out at an end portion of the sample injection area.

9. The biosensor of claim 1, wherein the spacer is made of a hydrophobic material to prevent the sample from running out at an end portion of the sample injection area.

10. The biosensor of claim 1, wherein the upper substrate further comprises a sample injection hole at a sample inlet of the sample injection area so that the sample can be easily injected.

11. The biosensor of claim 1, wherein the enzyme reactant layer comprises at least one kind of enzyme and at least one kind of electron-transfer mediator.

12. The biosensor of claim 11, wherein the enzyme is an oxidoreductase, a dehydrogenase, a transferase, or a hydrolase.

13. The biosensor of claim 1, wherein the electrode is made of carbon, graphite, platinum-carbon, silver, gold, palladium, or platinum.
